# EUROPEAN PATENT APPLICATION

(11) **EP 4 748 379 A1**
(43) Date of publication of application: **27.05.2026**
(21) Application number: 24855319.0
(22) Date of filing: 18.04.2024
(51) Int. Cl.: A61K 31/7072, A61P 5/50, A61P 3/00, A61P 3/04, A61P 3/10, A61P 3/06, A61P 1/16, A61P 9/10, A61P 9/12, C12N 5/071

(54) **USE OF URIDINE DIPHOSPHATE GLUCOSE IN TREATMENT OF ABNORMAL LIVER GLUCOLIPID METABOLISM**

(30) Priority: 18.08.2023 CN 202311047714
(71) Applicant: Institute of Basic Medical Sciences, Chinese Academy of Medical Sciences, Beijing 100005 (CN)
(72) Inventor: HUANG, Bo, Beijing 100005 (CN)
(74) Representative: Flesselles, Bruno F.G.
(86) International application number: PCT/CN2024/088588
(87) International publication number: WO 2025/039588

(57) **Abstract**

A use of uridine diphosphate glucose (UDPG) in treatment of abnormal liver glucolipid metabolism. Specifically, a use of UDPG or a pharmaceutical composition comprising same in the preparation of a drug for preventing and/or treating abnormal liver glucolipid metabolism, wherein the abnormal liver glucolipid metabolism is preferably nonalcoholic fatty liver disease, dyslipidemia and related cardiovascular diseases. Treatment using UDPG can reduce lipid synthesis and lipid synthesis related gene expression in the liver.

## Description

### FIELD OF THE INVENTION

The present invention relates to the field of biomedicine. Specifically, it relates to the use of uridine diphosphate glucose in treatment of abnormal liver glucose and lipid metabolism.

### BACKGROUND OF THE INVENTION

Glucose and lipid metabolic disorder (GLMD) refers to a class of comprehensive diseases characterized by dysregulation of glucose and lipid metabolism. It mainly includes a series of pathological symptoms such as obesity, Type II diabetes mellitus (T2DM), hypertension, dyslipidemia, non-alcoholic fatty liver disease (NAFLD), and atherosclerosis. With changes in modern lifestyles and improvements in living standards, glucose and lipid metabolic disorder has been proven to be one of the major chronic diseases causing global public and clinical problems. These diseases are closely interrelated and often coexist clinically, making their treatment highly challenging and they are widely acknowledged as a serious threat to human health. Studies indicate that although the pathogenesis of GLMD is not yet fully understood, it has been determined that various GLMDs share a common core risk factor: i.e., impaired glucose and lipid metabolism. The normal function of systemic glucose and lipid metabolism depends on coordinated interactions among multiple tissues and organs. However, the liver acts as the main metabolic control center. It is responsible for maintaining energy and nutritional homeostasis when physiological conditions change. The liver is the central hub of glucose and lipid metabolism, and its role in the occurrence and development of various glucose and lipid metabolic disorders cannot be ignored.

One of the main conditions of abnormal liver glucose and lipid metabolism is NAFLD. Data indicate that approximately 23% of the global population suffers from NAFLD, and this disease is one of the important causes of the global prevalence of liver disease. Data show that the NAFLD situation in developing countries is more severe. For example, in China, the prevalence of NAFLD is as high as 29.3%. We urgently need to address the severe challenges brought by the large-scale epidemic of NAFLD. However, to date, there are no FDA-approved drugs available for the treatment of NAFLD. Furthermore, the regulatory mechanisms of glucose and lipid metabolism in the liver have not been fully elucidated. Therefore, in-depth research into the pathogenesis of NAFLD is extremely important for the development of new drugs.

On the other hand, abnormal hepatic lipid metabolism can lead to elevated blood lipids, resulting in hypercholesterolemia and/or hypertriglyceridemia. It is well known that elevated blood lipids are directly associated with an increased risk of atherosclerotic cardiovascular disease.

Studies have shown that the liver is the central organ responsible for the synthesis, metabolism, storage, and redistribution of glucose and lipids. Within the liver, glucose and lipid metabolism are closely interconnected in multiple aspects. The inability of the body to degrade excessive lipids produced in the liver leads to excessive lipid deposition and the accumulation of harmful lipids, thereby causing liver injury. This is one of the main causes of NAFLD. Therefore, regulating lipid generation in the liver and inhibiting excessive lipid deposition and harmful lipid accumulation in the liver opens up new avenues for the prevention and treatment of NAFLD.

### SUMMARY OF THE INVENTION

The present invention is proposed to address the problems that there is currently no standard treatment method for NAFLD and no specific drug for NAFLD.

The inventors have unexpectedly found that uridine diphosphate glucose (UDPG) can effectively inhibit lipid synthesis in the liver, significantly reduce the content of triglycerides and cholesterol in the liver and/or serum, and inhibit fat accumulation in the liver.

Accordingly, in a first aspect, the present invention provides the use of uridine diphosphate glucose (UDPG) in the preparation of a medicament for preventing and/or treating a disease associated with abnormal liver glucose and lipid metabolism in a subject.

In another aspect, the present invention provides the use of a pharmaceutical composition comprising uridine diphosphate glucose in the preparation of a medicament for preventing and/or treating a disease associated with abnormal liver glucose and lipid metabolism in a subject, wherein the pharmaceutical composition further comprises a pharmaceutically acceptable carrier or excipient.

The disease associated with abnormal liver glucose and lipid metabolism includes, but is not limited to: insulin resistance, metabolic syndrome, obesity, Type II diabetes mellitus, dyslipidemia, non-alcoholic fatty liver disease (NAFLD), non-alcoholic steatohepatitis (NASH), coronary heart disease, hypertension, and atherosclerosis.

In a preferred embodiment, the abnormal liver glucose and lipid metabolism is NAFLD. In a preferred embodiment, the NAFLD is early-stage NAFLD. In a preferred embodiment, the NAFLD is high-fat diet-induced NAFLD.

In a specific embodiment, the dyslipidemia is hyperlipidemia, including but not limited to hypercholesterolemia and/or hypertriglyceridemia.

According to some embodiments of the present invention, the subject is a mammal, preferably a human.

According to some embodiments of the present invention, the medicament is formulated as a dosage form for oral or parenteral administration, preferably formulated as a dosage form for oral, intradermal, subcutaneous, intraperitoneal, intrahepatic, or intravenous administration.

According to some embodiments of the present invention, the uridine diphosphate glucose reduces lipid synthesis in the liver of the subject. Preferably, the uridine diphosphate glucose reduces the synthesis of triglycerides and/or cholesterol in the liver.

According to some embodiments of the present invention, the uridine diphosphate glucose reduces the content of triglycerides and/or total cholesterol in the serum of the subject.

According to some embodiments of the present invention, the subject suffers from or is suspected of suffering from abnormal liver glucose and lipid metabolism or is at risk of abnormal liver glucose and lipid metabolism.

In another aspect, the present invention provides a method for preventing and/or treating a disease associated with abnormal liver glucose and lipid metabolism in a subject, wherein the method comprises administering a therapeutically effective amount of uridine diphosphate glucose to the subject.

In a specific embodiment, the present invention provides a method for preventing and/or treating a disease associated with abnormal liver glucose and lipid metabolism in a subject, wherein the method comprises administering a therapeutically effective amount of uridine diphosphate glucose to the subject; the disease associated with abnormal liver glucose and lipid metabolism includes, but is not limited to, insulin resistance, metabolic syndrome, obesity, Type II diabetes mellitus, dyslipidemia, NAFLD, NASH, coronary heart disease, hypertension, and atherosclerosis.

In a specific embodiment, a therapeutically effective amount of uridine diphosphate glucose is administered to the subject orally or parenterally. Preferably, a therapeutically effective amount of uridine diphosphate glucose is administered to the subject orally, intradermally, subcutaneously, intraperitoneally, intrahepatically, or intravenously.

In another aspect, the present invention provides an *in vitro* method for reducing lipid synthesis in hepatocytes or liver organoids, wherein the method comprises the step of contacting the hepatocytes or liver organoids with uridine diphosphate glucose *in vitro.*

In a specific embodiment, the present invention provides an *in vitro* method for reducing lipid synthesis in hepatocytes or liver organoids, wherein the method comprises the following steps:
1) culturing hepatocytes or liver organoids *in vitro* under conditions suitable for growth; and
2) contacting the hepatocytes or liver organoids cultured *in vitro* in step 1) with uridine diphosphate glucose *in vitro.*

Through extensive experimental research, the inventors found that UDPG can effectively inhibit lipid synthesis in the liver and is a novel therapeutic strategy for NAFLD. Glycogen synthesis in hepatocytes is shunted to the Golgi apparatus by the transport of the intermediate metabolite UDPG via solute carrier 35 (SLC35). In the Golgi apparatus, UDPG inhibits the activity of Binding Site-1 protease (S1P) and inhibits the S1P-mediated cleavage of sterol regulatory element-binding protein (SREBP), thereby inhibiting lipid synthesis within the liver. *In vivo* studies found that, compared with the control group, UDPG treatment alleviated hepatic steatosis in a fatty liver mouse model and inhibited hepatic lipid synthesis in liver organoids and NAFLD patients.

Compared to other drugs in clinical trials, UDPG has the following significant advantages: (1) UDPG is naturally present in human peripheral blood and can be effectively administered via intravenous injection; (2) UDPG participates in the conversion of monosaccharides to glucose in the human body and is therefore friendly to patients; (3) UDPG can be taken up by hepatocytes, ensuring the feasibility of its regulation of hepatic lipid synthesis; and (4) UDPG may promote glycogen metabolism, which contributes to the clearance of reactive oxygen species (ROS) by regulating the pentose phosphate pathway.

### DESCRIPTION OF DRAWINGS

Figure 1A and Figure 1B show that UDPG can reduce lipid synthesis and the expression of lipid synthesis-related genes in mouse hepatocytes.
Figure 2A to Figure 2F show that UDPG can reduce the body weight of fatty liver mice and reduce lipid and cholesterol synthesis in the liver and serum.
Figure 3A to Figure 3C show that the UDPG level in hepatocytes of NAFLD patients is low, and UDPG can reduce lipid synthesis and the expression of lipid synthesis-related genes in adult hepatocytes.
Figure 4A to Figure 4C show that UDPG can reduce lipid synthesis in human liver organoids.

### DETAILED DESCRIPTION OF THE INVENTION

To prevent and/or treat diseases associated with abnormal liver glucose and lipid metabolism, the medicament or pharmaceutical composition of the present invention may be used alone or in combination with surgery, endocrine therapy, drug therapy, and methods using biological response modifiers.

In some specific embodiments, the medicament or pharmaceutical composition of the present invention comprises uridine diphosphate glucose (UDPG) as an active ingredient, and optionally comprises a pharmaceutically acceptable carrier or excipient.

Uridine diphosphate glucose, abbreviated as UDPG, is a type of nucleotide sugar widely distributed in microbial, plant, and animal cells. It is used as a glucose donor during the biosynthesis of various glycosides, oligosaccharides, and polysaccharides in organisms. In addition, UDPG plays a central role in carbohydrate metabolism as an important intermediate during the interconversion of monosaccharides or the formation of uronic acids.

In other specific embodiments, UDPG is used in combination with one or more other treatment methods or therapeutic agents. The treatment methods are preferably chemotherapy or targeted therapy, and the therapeutic agents are preferably other agents for preventing and/or treating abnormal liver glucose and lipid metabolism.

The medicament or pharmaceutical composition of the present invention may conveniently be presented in unit dosage form. The medicament or pharmaceutical composition of the present invention may be formulated into any appropriate dosage form, such as, but not limited to, injections, tablets, capsules, gels, etc. The medicament or pharmaceutical composition of the present invention may also be formulated as suspensions in aqueous, non-aqueous, or mixed media. The medicament or pharmaceutical composition of the present invention includes, but is not limited to, solutions, emulsions, foams, and liposome-containing formulations. The pharmaceutical composition of the present invention may include one or more penetration enhancers, carriers, or excipients.

In some embodiments, the disease to be intervened by the medicament or pharmaceutical composition of the present invention is selected from any one of the following or a combination thereof: insulin resistance, metabolic syndrome, obesity, Type II diabetes mellitus, dyslipidemia, non-alcoholic fatty liver disease, non-alcoholic steatohepatitis, coronary heart disease, hypertension, and atherosclerosis.

In some specific embodiments, the medicament or pharmaceutical composition of the present invention is employed to prevent and/or treat NAFLD.

The term "non-alcoholic fatty liver disease (NAFLD)" as used herein is a common liver disease. It is the manifestation of metabolic syndrome in the liver and is a concentrated reflection of related diseases such as obesity, insulin resistance, Type II diabetes mellitus, hypertension, and hyperlipidemia.

The term "dyslipidemia" as used herein refers to a relatively common class of diseases. It is a metabolic abnormality of lipoproteins in the human body, mainly manifested as elevated total cholesterol (TC), low-density lipoprotein cholesterol (LDL-C), and triglycerides (TG) and/or decreased high-density lipoprotein cholesterol (HDL-C) in serum. Dyslipidemia is one of the important factors leading to atherosclerosis and is an independent risk factor for coronary heart disease and ischemic stroke.

The term "treatment" refers to eliminating the disease, arresting disease progression, slowing disease progression, reducing the duration of one or more symptoms associated with the disease, improving or reversing at least one measurable parameter associated with the disease, or increasing the survival rate of a subject suffering from the disease.

In some embodiments, UDPG or a pharmaceutical composition comprising the same is administered to a subject in a therapeutically effective amount.

The term "therapeutically effective amount" may refer to the amount of the active ingredient or pharmaceutical composition that induces a biological or medical response in an animal or human that is considered by a researcher, veterinarian, physician, or other clinician. Such an amount may include the amount of the active ingredient or pharmaceutical composition used to induce alleviation of the disease or condition to be treated. It will be apparent to those skilled in the art that the therapeutically effective dose and frequency of administration of the active ingredients of the present invention may vary depending on the desired effect. The term "subject" refers to an animal, preferably a mammal. According to specific embodiments, the subject is a mammal, including for example camels, donkeys, zebras, cows, pigs, horses, goats, sheep, cats, dogs, rats, rabbits, guinea pigs, mice, and primates (*e.g*., humans). In specific embodiments, the subject is a human. In specific embodiments, the subject is a person susceptible to, suspected of suffering from, or having already suffered from abnormal liver glucose and lipid metabolism, wherein the abnormal liver glucose and lipid metabolism is preferably NAFLD. The term "administering" may refer to providing a predetermined substance to a subject by any appropriate method.

The administration amount or intake amount can be administered in various administration doses and methods. The composition is distributed according to the subject's body weight, age, gender, health status, diet, administration time, administration method, excretion rate, and disease severity, for example, once a day or multiple times a day.

In the present invention, the pharmaceutical composition of the present invention can be administered via any general route, as long as it can reach the target tissue. Administration can be performed orally, intraperitoneally, intravenously, intramuscularly, subcutaneously, intradermally, intranasally, intrapulmonarily, rectally, intracavitarily, intraperitoneally, and intrathecally, but is not limited thereto. In a preferred embodiment, the medicament is administered parenterally, preferably via intravenous injection.

### Examples

Experimental Materials Male wild-type C57BL/6 mice were purchased from the Institute of Laboratory Animal Sciences, Chinese Academy of Medical Sciences (Beijing, China). These animals were housed under specific pathogen-free conditions in the animal facility of the Chinese Academy of Medical Sciences. All studies involving mice were approved by the Animal Care and Use Committee of the Chinese Academy of Medical Sciences (ACUC-A02-2020-009).

All research protocols involving human liver tissue samples were approved by the First Affiliated Hospital of Zhengzhou University. All protocols complied with the Declaration of Helsinki and the Declaration of the World Medical Association. Informed consent forms were signed by all individuals or their family members.

### Example 1. UDPG reduced lipid synthesis in mouse hepatocytes.

### 1. Experimental Process

Primary hepatocytes were isolated from mice by a two-step collagenase perfusion method. The cells were plated into a 6-well plate at 1.6×10⁵ cells per well. After the cells adhered, hepatocytes in the experimental groups were treated with 100 µM and 200 µM UDPG. Hepatocytes in the control group were treated with the same concentration of G1P. After 48 hours of treatment, the medium was discarded, and the cells were washed twice with PBS. The PBS was completely discarded after the last wash. The cells were scraped, and then TG levels were determined by the cuvette method.

Primary hepatocytes were isolated from mice by a two-step collagenase perfusion method. The cells were plated into a 6-well plate at 1.6×10⁵ cells per well. After the cells adhered, the hepatocytes were treated with 100 µM and 200 µM UDPG for 48 hours. The medium was discarded, and the cells were washed twice with PBS. The PBS was completely discarded after the last wash. 1 mL of TRIZOL was added to each well. Cellular RNA was extracted and reverse transcription was performed. The expression of lipid synthesis-related genes *Acaca*, *Fasn,* and *Scd1* was analyzed using qPCR.

### 2. Experimental Results

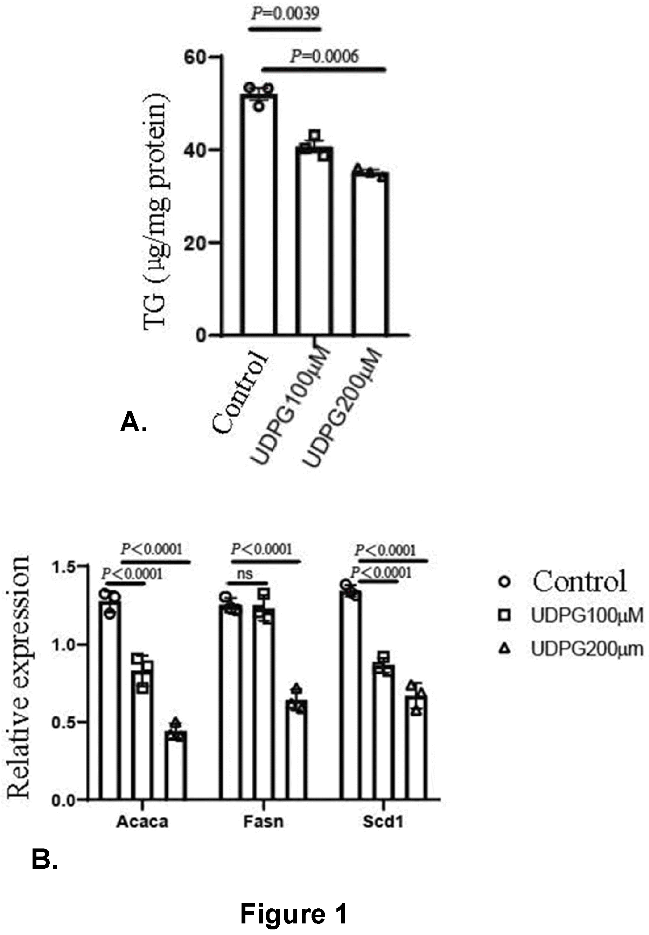

Compared with the control group, TG levels decreased after treating hepatocytes with 100 µM and 200 µM UDPG, indicating that lipid synthesis was reduced (Figure 1A) . qPCR analysis showed that UDPG treatment reduced the expression of lipid synthesis-related genes *Acaca*, *Fasn,* and *Scd1* in hepatocytes (Figure 1B).

### Example 2. UDPG reduced the body weight of fatty liver mice and reduced lipid and cholesterol synthesis in the liver and serum.

### 1. Experimental Process

C57BL/6 mice were divided into three groups (n=6 per group). One group was fed a normal diet (ND), and the other two groups were fed a high-fat diet (HFD). After 8 weeks of feeding, one group of HFD mice was intraperitoneally injected with UDPG (8 mg/kg) (HFD+UDPG group). The other group of HFD mice and the ND mice were intraperitoneally injected with an equal volume of PBS solution (ND+Vehicle group, HFD+Vehicle group). Injections were administered once daily for 8 consecutive weeks. The body weight of the mice was weighed daily. After 8 weeks of injection, the mice were sacrificed. Liver tissue and blood were collected, and serum was separated. A portion of the liver tissue and serum was taken, and TG and TC levels in the liver tissue and blood were determined using colorimetric methods. A portion of the liver tissue was fixed in 4% paraformaldehyde for 48 hours, embedded in paraffin, and made into pathological sections. Subsequently, Hematoxylin and Eosin (H&E) staining and Oil Red O staining were performed, and the sections were observed under a microscope.

### 2. Experimental Results

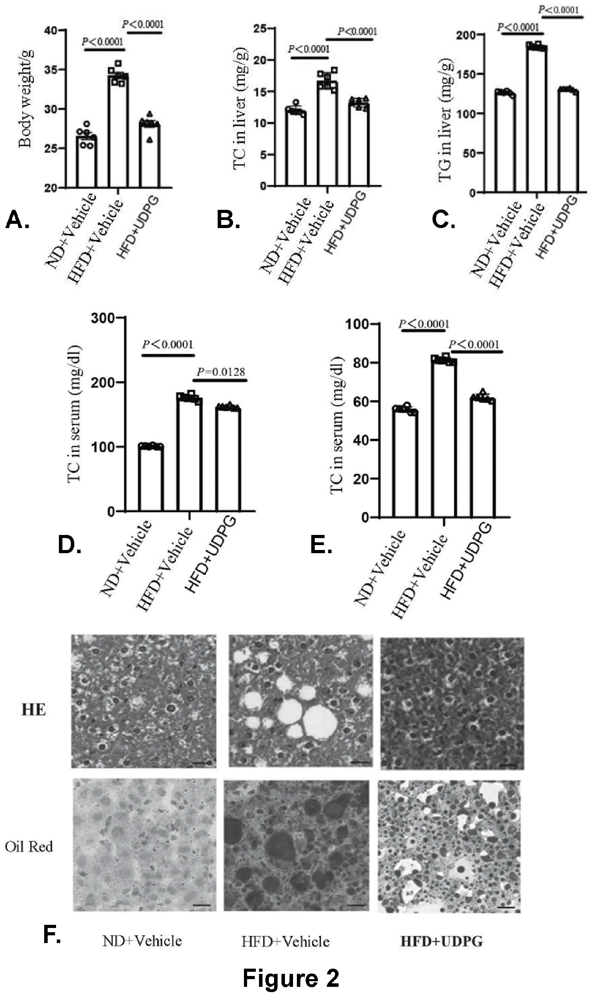

The body weight of mice in the HFD+UDPG group was lower than that in the HFD+Vehicle group (Figure 2A). The levels of TG and TC in the liver and serum were significantly lower than those in the HFD+Vehicle group (Figure 2B to Figure 2E), indicating that the amount of lipid and cholesterol synthesis was lower. The livers of mice in the HFD+Vehicle group showed vesicular steatosis and obvious lipid droplets, whereas no obvious degeneration was observed in the livers of mice in the HFD+UDPG group, and the lipid droplets were significantly smaller than those in the HFD+Vehicle group (Figure 2F).

### Example 3. UDPG inhibited lipid synthesis in hepatocytes of NAFLD patients.

### 1. Experimental Process

Primary hepatocytes were isolated from liver tissues of NAFLD patients by a two-step collagenase perfusion method. The cell suspension was collected and plated into a 6-well plate at 1.6×10⁵ cells per well. After the cells adhered, they were washed twice with PBS. The PBS was completely discarded after the last wash. The hepatocytes were scraped, and the UDPG levels in the patients' liver tissues were detected.

Primary hepatocytes were isolated from adult liver tissues by a two-step collagenase perfusion method. The cell suspension was collected and plated into a 6-well plate at 1.6×10⁵ cells per well. After the cells adhered, 200 µM UDPG was added to the experimental group for pretreatment, and an equal amount of PBS was added to the control group. Subsequently, 400 µM free fatty acid (FFA) was added for treatment for 48 hours. The medium was discarded, and the cells were washed twice with PBS. The PBS was completely discarded after the last wash. The hepatocytes were scraped. A portion of the cells was taken to detect TG levels in the two groups by the cuvette method. TRIZOL was added to a portion of the cells to extract cellular RNA, which was then subjected to reverse transcription. The expression of lipid synthesis genes *Acaca*, *Fasn,* and *Scd1* was analyzed using qPCR.

### 2. Experimental Results

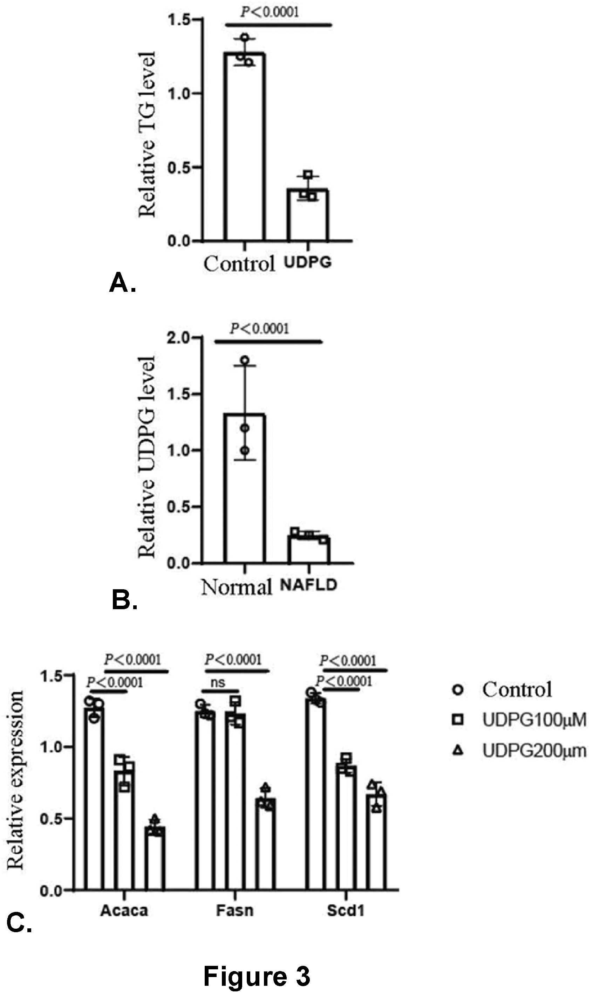

The UDPG levels in the liver tissues of NAFLD patients were far lower than those in normal liver tissues (Figure 3A). Adult hepatocytes were treated with FFA. The TG levels in cells pretreated with UDPG were significantly lower than those in the control group (Figure 3B), indicating that the amount of lipid synthesis was lower. The expression of lipid synthesis-related genes *Acaca*, *Fasn,* and *Scd1* was also significantly lower than that in the control group (Figure 3C).

### Example 4. UDPG reduced lipid synthesis in liver organoids.

### 1. Experimental Process

Primary hepatocytes were isolated from adult liver tissues by a two-step collagenase perfusion method. The cell suspension was collected. Matrigel gel prepared in advance was mixed with the cell suspension at a ratio of 1:3, and the cell density was adjusted according to the cell count results to achieve 1×10⁴ hepatocytes/well in a 12-well plate. Subsequently, 20 µL of the mixture with various concentrations was dropped into the center of the well to form a sphere. After a short period of standing to allow for slight solidification, the plate was carefully inverted and placed in a cell incubator. After 1 hour, 500 µL of medium was added to each well. The growth and morphological changes of the liver organoids were observed daily, and the medium was changed every 2 to 3 days.

After the liver organoids grew for 2 to 3 days, 200 µM UDPG was added to the experimental group for pretreatment, and an equal amount of PBS was added to the control group. Subsequently, 400 µM oleic acid (OA) was added for the treatment of 48 hours. The culture medium was discarded, and the liver organoids were washed twice with PBS. The PBS was completely discarded after the last wash.

For TG measurement, 200 µL of cell recovery solution was added to each well. The matrix gel in the well was disrupted by pipetting and then aspirated and collected into an EP tube. It was incubated on ice until the matrix gel completely melted. The mixture was centrifuged at 4°C and 800 rpm for 5 min, and the supernatant was discarded. 500 µL of trypsin was added for digestion, and the mixture was incubated at 37°C. Observation was performed under a microscope, and digestion was terminated when the clonal spheres were observed to disperse into single cells. The cells were collected by centrifugation at 800 rpm for 5 min.

For morphological observation, liver organoids with as intact morphology as possible were obtained by digestion from the matrix gel and collected into an EP tube. The liver organoids were fixed with 4% paraformaldehyde (PFA) on ice for 1 h, then washed with cold PBS, and centrifuged at 4°C and 1500 rpm for 5 min. This procedure was repeated 2 to 3 times. Subsequently, the cells were resuspended in 4% PFA and fixed at 4°C overnight. After fixation was completed, centrifugation was performed, and the 4% PFA was discarded. 2% low melting point agarose was prepared using sterile PBS and heated in a microwave oven until completely melted. The liver organoids were resuspended with the liquid agarose while it was still warm, and immediately added to a ring mold placed on ice. It was placed on ice for 1 h until the agarose completely solidified. The solidified agarose block was then subjected to paraffin embedding, sectioning, and Bodipy staining.

### 2. Experimental Results

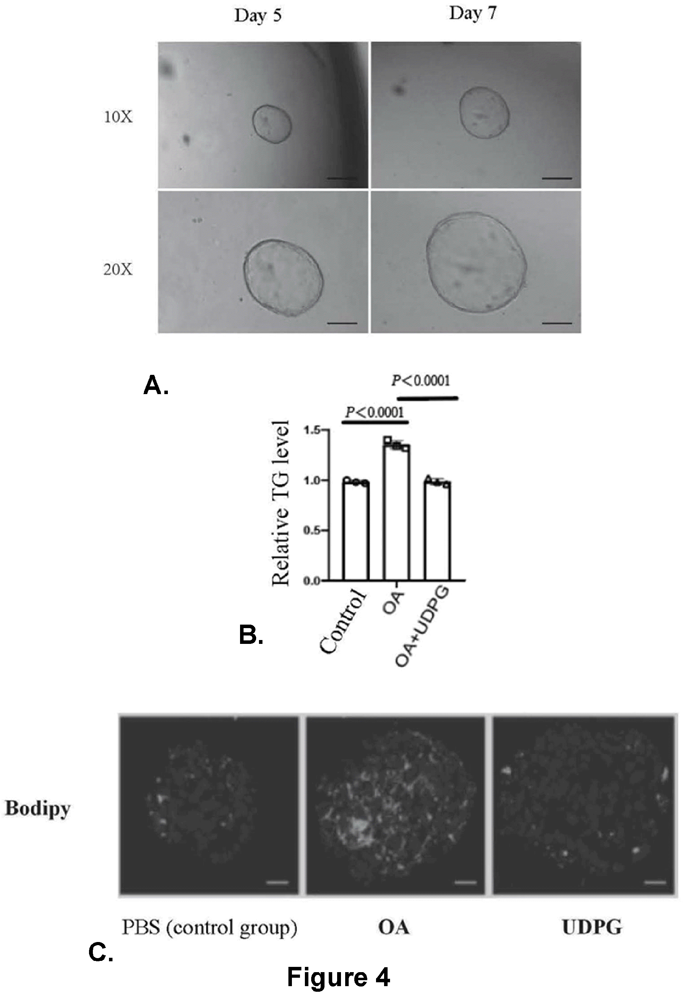

Primary hepatocytes expanded rapidly in the 3D culture environment. After 3 days of induction culture, they expanded from single cells to clonal spheres with a diameter of 100 µm, and formed organoids with a cystic structure of 300 µm on Day 7 (Figure 4A). The organoids were treated with oleic acid. The TG levels in the UDPG pretreated organoids were significantly lower than those in the control group (Figure 4B), indicating that the amount of lipid synthesis was lower.

In the control group, obvious lipid particles appeared in the Bodipy stained sections of the organoids, while the lipid particles in the UDPG group were significantly decreased (Figure 4C).

The results of the above examples indicate that:
UDPG effectively inhibits hepatic lipid synthesis and represents a novel therapeutic strategy for NAFLD.

*In vivo* studies show that, compared with the blank control group, UDPG treatment significantly reduces hepatic lipid synthesis in animal models and alleviates the degree of hepatic steatosis.

Compared with other drugs in clinical trials, UDPG is naturally present in human peripheral blood, can be effectively administered via intravenous injection, is readily taken up by hepatocytes, thus ensuring the feasibility of its regulation of hepatic lipid synthesis, exhibits high safety with no apparent toxic side effects, and is suitable for direct use in humans.

## Claims

1. Use of uridine diphosphate glucose in the preparation of a medicament for preventing and/or treating a disease associated with abnormal liver glucose and lipid metabolism in a subject.

2. Use of a pharmaceutical composition comprising uridine diphosphate glucose in the preparation of a medicament for preventing and/or treating a disease associated with abnormal liver glucose and lipid metabolism in a subject, wherein the pharmaceutical composition further comprises a pharmaceutically acceptable carrier or excipient.

3. The use according to claim 1 or 2, wherein the disease associated with abnormal liver glucose and lipid metabolism is selected from the group consisting of: insulin resistance, metabolic syndrome, obesity, Type II diabetes mellitus, dyslipidemia, non-alcoholic fatty liver disease, non-alcoholic steatohepatitis, coronary heart disease, hypertension, and atherosclerosis.

4. The use according to claim 1 or 2, wherein the dyslipidemia is hyperlipidemia, preferably hypercholesterolemia and/or hypertriglyceridemia.

5. The use according to claim 1 or 2, wherein the subject is a mammal, preferably a human.

6. The use according to claim 1 or 2, wherein the medicament is formulated as a dosage form for oral or parenteral administration, preferably formulated as a dosage form for oral, intradermal, subcutaneous, intraperitoneal, intrahepatic, or intravenous administration.

7. The use according to claim 1 or 2, wherein the uridine diphosphate glucose reduces hepatic lipid synthesis in the subject, preferably, the uridine diphosphate glucose reduces the hepatic synthesis of triglycerides and/or cholesterol.

8. The use according to claim 1 or 2, wherein the uridine diphosphate glucose reduces the serum content of triglycerides and/or total cholesterol in the subject.

9. The use according to claim 1 or 2, wherein the subject suffers from or is suspected of suffering from abnormal liver glucose and lipid metabolism or is at risk of abnormal liver glucose and lipid metabolism.

10. An *in vitro* method for reducing lipid synthesis in hepatocytes or liver organoids, wherein the method comprises the step of contacting the hepatocytes or liver organoids with uridine diphosphate glucose *in vitro.*
